# EUROPEAN PATENT APPLICATION

(11) **EP 2 253 224 A1**
(43) Date of publication of application: **24.11.2010**
(21) Application number: 10163078.8
(22) Date of filing: 18.05.2010
(51) Int. Cl.: A23G 4/06, A61K 9/00, A61K 47/02

(54) **Ondansetron film compositions**

(30) Priority: 19.05.2009 US 468434
(71) Applicant: MonoSol RX LLC, Portage, IN 46368 (US)
(72) Inventor: Myers, Gary L., KINGSPORT, TN 37660 (US); Hariharan, Madhusudan, MUNSTER, IN 46321 (US); Sanghvi, Pradeep, SCHERERVILLE, IN 43675 (US)
(74) Representative: Raggers, René John

(57) **Abstract**

The present invention relates to products and methods of making products having a taste masked active component. In particular, the present invention relates to taste-masked film dosage forms including at least one active component and a slow dissolving basic composition.

## Description

### Field of the Invention

The present invention relates to film formulations including at least one active component. More particularly, the invention relates to products and methods of making products that acceptably taste-mask the active component. The active component may be a weak base, such as ondansetron.

### Background of the Invention

Administration of active components, particularly pharmaceutical compositions, may be achieved in many different forms. Since such active components typically have a bitter, foul taste, administration of active components may be achieved by swallowing tablets or other dosage forms, which limits the time that the active component is present in the mouth of the user.

However, it may be desired to administer active components through an orally dissolvable film dosage form, which is placed in the mouth and allowed to dissolve, thereby releasing the active component. Such film dosage forms are beneficial for several reasons, including ease of administration, particularly to those individuals who have difficulty swallowing pills or tablets. In addition, film dosage forms are useful to protect against abuse of the active component. Pills and tablets may be held in the mouth of the user without swallowing, and then removed at a later date and abused. Film dosages, on the other hand, may have a high mucoadhesivity, whereby the film adheres inside the mouth of the user, limiting or altogether preventing its removal from the user.

When the film dosage dissolves in the mouth of the user, it releases the active component into the mouth of the user. The active component is then swallowed. Unfortunately, while the active component is released in the mouth, the user is subjected to the taste of the active component. The taste is likely to be extremely displeasing to the user. Methods of taste masking the active component, while useful, have not been able to sufficiently block the foul taste of the active. Traditionally, taste-masking is achieved through various means of flavoring the composition to cover up the taste of the active, complexation of the active with an ion exchange resin, thereby binding the drug, or by coating the active component with a polymer coating to act as a barrier to the taste buds. However, each of these currently-used taste-masking methods not only adds additional cost to the active but also is often incapable of effectively masking the foul taste of the active.

There is thus a need for an active component based film composition that effectively masks the foul taste of the active component and which solves the problems associated with the prior art.

### Summary of the Invention

In one embodiment of the present invention, there is provided a self-supporting film dosage composition including: a polymer, an active component and at least one slow-dissolving alkaline component.

In another embodiment, there is provided a self-supporting film dosage composition including: an active component in an amount of about 10-15% by weight of the composition; at least one slow-dissolving alkaline component in an amount of about 1-10% by weight of the composition; and a polymeric matrix in an amount of about 75-89% by weight of the composition.

In still other embodiments of the present invention, there is provided a method of making a self-supporting film dosage composition including the steps of: providing a film-forming polymeric matrix; providing at least one active component; providing a slow-dissolving alkaline component; providing a fast dissolving alkaline component; combining the film-forming polymeric matrix, at least one active and the slow-dissolving and fast-dissolving basic component to form an active matrix; and drying the active matrix to form the self-supporting film dosage composition.

### Detailed Description

The present invention provides improved methods and products for oral administration of active components. In some embodiments, the active component (or "drug") is delivered to the user via an orally dissolvable film strip. The present invention seeks to reduce or altogether eliminate any foul or bitter taste associated with the drug, particularly when administered in a dissolving film dosage.

The present invention provides a pharmaceutical composition in the form of a film for oral administration, including a composition having a uniformly distributed combination of a polymer, a polar solvent, a sweetening agent, and a pharmaceutically active or bioeffecting agent. It will be understood that the term "film" includes thin films and sheets, in any shape, including rectangular, square, or other desired shape. The films described herein may be any desired thickness and size such that it may be placed into the oral cavity of the user. For example, the films may have a relatively thin thickness of from about 0.1 to about 10 mils, or they may have a somewhat thicker thickness of from about 10 to about 30 mils. For some films, the thickness may be even larger, i.e., greater than about 30 mils. In addition, the term "film" includes single-layer compositions as well as multi-layer compositions, such as laminated films. The composition in its dried film form maintains a uniform distribution of components through the application of controlled drying of the film.

The film dosage composition preferably includes a polymeric carrier matrix, also referred to as a wet film-forming matrix. Any desired polymeric carrier matrix may be used, provided that it is orally dissolvable and is suitable for human ingestion. The orally consumable films are preferably fast-dissolving or moderate-dissolving in the oral cavity and particularly suitable for delivery of actives. In general if a drug is coated to modulate the release of the drug, to some extent the drug is taste-masked, however residual amounts of 'free' (uncoated) drug that are present in the formulation due to incomplete coating or due to the inclusion of uncoated drug as a loading dose may contribute to bitter taste that can be masked by the inclusion of the alkaline components discussed above.

The films used in the pharmaceutical products may be produced by a combination of at least one polymer and a polar solvent, optionally including other fillers known in the art. The solvent may be water, a polar organic solvent including, but not limited to, ethanol, isopropanol, acetone, or any combination thereof. The film may be prepared by utilizing a selected casting or deposition method and a controlled drying process. For example, the film may be prepared through controlled drying processes, which include application of heat and/or radiation energy to the wet film matrix to form a visco-elastic structure in a short period of time (such as less than 10 minutes), thereby controlling the uniformity of content of the film. Such processes are described in more detail in commonly assigned U.S. Patent Nos. 7,425,292 and 7,357,891, the contents of which are incorporated herein by reference in their entirety. Alternatively, the films may be extruded as described in commonly assigned U.S. Application No. 10/856,176, filed on May 28, 2004, and published as U.S. Patent Publication No. 2005/0037055 A1, the contents of which are incorporated herein by reference in their entirety.

The polymer(s) included in the films may be water-soluble, water-swellable, water-insoluble, pH sensitive or a combination of one or more either water-soluble, water-swellable, pH sensitive or water-insoluble polymers. As used herein the phrase "water-soluble polymer" and variants thereof refer to a polymer that is at least partially soluble in water, and desirably fully or predominantly soluble in water, or absorbs water. Polymers that absorb water are often referred to as being water-swellable polymers. The materials useful with the present invention may be water-soluble, pH sensitive or water-swellable at room temperature and other temperatures, such as temperatures exceeding room temperature. Moreover, the materials may be water-soluble or water-swellable at pressures less than atmospheric pressure. Desirably, the water-soluble polymers are water-soluble or water-swellable having at least 20 percent by weight water uptake. Water-swellable polymers having a 25 or greater percent by weight water uptake are also useful. In some embodiments, films formed from such water-soluble polymers may be sufficiently water-soluble to be dissolvable upon contact with bodily fluids. The polymer may include cellulose or a cellulose derivative

Specific examples of useful polymers include, but are not limited to, polyethylene oxide, pullulan, hydroxypropylmethyl cellulose, hydroxyethyl cellulose, hydroxypropyl cellulose, polyvinyl pyrrolidone, carboxymethyl cellulose, polydextrose, polyvinyl alcohol, sodium alginate, propylene glycol alginate, carrageenan, polyethylene glycol, xanthan gum, tragancanth gum, guar gum, acacia gum, arabic gum, polyacrylic acid, methylmethacrylate copolymer, copolymers of acrylic acid and alkyl acrylate (available as Pemulen® polymers), carboxyvinyl copolymers, starch, gelatin, pectin, and combinations thereof. Specific examples of useful water-insoluble polymers include, but are not limited to, ethyl cellulose, hydroxypropyl ethyl cellulose, cellulose acetate phthalate, hydroxypropyl methyl cellulose phthalate and combinations thereof.

Other polymers useful for incorporation into the films include biodegradable polymers, copolymers, block polymers and combinations thereof. Among the known useful polymers or polymer classes which meet the above criteria are: poly(glycolic acid) (PGA), poly(lactic acid) (PLA), polydioxanoes, polyoxalates, poly(α-esters), polyanhydrides, polyacetates, polycaprolactones, poly(orthoesters), polyamino acids, polyaminocarbonates, polyurethanes, polycarbonates, polyamides, poly(alkyl cyanoacrylates), and mixtures and copolymers thereof. Additional useful polymers include, stereopolymers of L- and D-lactic acid, copolymers of bis(p-carboxyphenoxy) propane acid and sebacic acid, sebacic acid copolymers, copolymers of caprolactone, poly(lactic acid)/poly(glycolic acid)/polyethyleneglycol copolymers, copolymers of polyurethane and (poly(lactic acid), copolymers of polyurethane and poly(lactic acid), copolymers of α-amino acids, copolymers of α-amino acids and caproic acid, copolymers of α-benzyl glutamate and polyethylene glycol, copolymers of succinate and poly(glycols), polyphosphazene, polyhydroxy-alkanoates and mixtures thereof. Binary and ternary systems are contemplated.

Specific examples of useful water insoluble polymers include, but are not limited to, ethyl cellulose, hydroxypropyl ethyl cellulose, cellulose acetate phthalate, hydroxypropyl methyl cellulose phthalate, acrylic polymers, vinyl acetate, sodium sulphonated polyesters, carboxylated acrylics, trimethylpentanediol/adipic acid/glycerin cross polymer, polyglycerol-2-diisostearate/IPDI copolymer, carboxylated vinyl acetate copolymer, vinylpyrrolicone/vinyl acetate/alkylaminoacrylate terpolymers, vinylpyrrolidone/vinyl acetate copolymer, and combinations thereof.

Other specific polymers useful include those marketed under the Medisorb and Biodel trademarks. The Medisorb materials are marketed by the Dupont Company of Wilmington, Delaware and are generically identified as a "lactide/glycolide co-polymer" containing "propanoic acid, 2-hydroxy-polymer with hydroxy-polymer with hydroxyacetic acid." Four such polymers include lactide/glycolide 100L, believed to be 100% lactide having a melting point within the range of 338°-347°F (170°-175°C); lactide/glycolide 100L, believed to be 100% glycolide having a melting point within the range of 437°-455°F (225°-235°C); lactide/glycolide 85/15, believed to be 85% lactide and 15% glycolide with a melting point within the range of 338°-347°F (170°-175° C); and lactide/glycolide 50/50, believed to be a copolymer of 50% lactide and 50% glycolide with a melting point within the range of 338°-347°F (170°-175°C). The Biodel materials represent a family of various polyanhydrides which differ chemically.

Although a variety of different polymers may be used, it is desired to select polymers that provide mucoadhesive properties to the film, as well as a desired dissolution and/or disintegration rate. In particular, the time period for which it is desired to maintain the film in contact with the mucosal tissue depends on the type of active contained in the second delivery vehicle. Some actives may only require a few minutes for delivery through the mucosal tissue, whereas other actives may require up to several hours or even longer. Accordingly, in some embodiments, one or more water-soluble polymers, as described above, may be used to form the film. In other embodiments, however, it may be desirable to use combinations of water-soluble polymers and polymers that are water-swellable, water- insoluble and/or biodegradable, as provided above. The inclusion of one or more polymers that are water-swellable, water-insoluble and/or biodegradable may provide films with slower dissolution or disintegration rates than films formed from water-soluble polymers alone. As such, the film may adhere to the mucosal tissue for longer periods or time, such as up to several hours, which may be desirable for delivery of certain active ingredients.

Oral dissolving films generally fall into three main classes: fast dissolving, moderate dissolving and slow dissolving. Fast dissolving films generally dissolve in about 1 second to about 30 seconds. Moderate dissolving films generally dissolve in about 1 to about 30 minutes, and slow dissolving films generally dissolve in more than 30 minutes. Fast dissolving films may consist of low molecular weight hydrophilic polymers (i.e., polymers having a molecular weight between about 1,000 to 9,000, or generally having a molecular weight below 200,000). In contrast, slow dissolving films generally have high molecular weight polymers (i.e., having a molecular weight in the millions).

Moderate dissolving films tend to fall in between the fast and slow dissolving films. Moderate dissolving films dissolve rather quickly, but also have a good level of mucoadhesion. Moderate films are also flexible, quickly wettable, and are typically nonirritating to the user. For the instant invention, it is preferable to use films that fall between the categories of fast dissolving and moderate dissolving. Such films provide a quick enough dissolution rate (between about 1 minute and about 5 minutes), while providing an acceptable mucoadhesion level such that the film is not easily removable once it is placed in the oral cavity of the user.

Desirably, the individual film dosage has a small size that is between about 0.5-2.0 inch by about 0.5-1.5 inch. Preferably, the film dosage is about 0.75 inches x 0.5 inches and more preferably about 1.25" to about 1.5". The film dosage should have good adhesion when placed in the buccal cavity or in the sublingual region of the user. Further, the film dosage should disperse and dissolve at a moderate rate, that is, between about 1 minute to about 30 minutes, and most desirably between about 10 minutes and about 20 minutes. In some embodiments, however, it may be desired to allow the individual film dosage to dissolve slower, over a period of longer than about 30 minutes. In such slow dissolving embodiments, it is preferable that the film dosage has strong mucoadhesion properties.

The films of the present invention may include more than one polymer. For instance, in some embodiments, the films may include polyethylene oxide alone or in combination with a second polymer component. In some embodiments, the films may include polymers other than polyethylene oxide. The second polymer may be another water-soluble polymer, a water-swellable polymer, a water-insoluble polymer, a biodegradable polymer or any combination thereof. Suitable water-soluble polymers include, without limitation, any of those provided above.

In accordance with some embodiments, polyethylene oxide may range from about 10% to 100% by weight in the polymer component, more specifically about 15% to about 70% by weight, and even more specifically about 20% to about 50% by weight. In one particular embodiment, the composition may include polyethylene oxide in an amount of about 14%. In some embodiments, one or more water-swellable, water-insoluble and/or biodegradable polymers also may be included in the polyethylene oxide-based film. In one particular embodiment, the composition may include a second polymer component, such as METHOCEL, in addition to the polyethylene oxide. Any of the water-swellable, water-insoluble or biodegradable polymers provided above may be employed. This second polymer component may be employed in amounts of about 0% to about 80% by weight in the polymer component, more specifically about 15% to about 70% by weight, and even more specifically about 25% to about 50% by weight. In one particular embodiment, the composition may include a second polymeric component in an amount of about 26%.

The molecular weight of the polyethylene oxide also may be varied. In some embodiments, high molecular weight polyethylene oxide, such as about 4 million, may be desired to increase mucoadhesivity of the film. In some other embodiments, the molecular weight may range from about 100,000 to 900,000, more specifically from about 100,000 to 600,000, and even more specifically from about 100,000 to 300,000.

In some embodiments, it may be desirable to combine high molecular weight (600,000 to 900,000) with low molecular weight (100,000 to 300,000) polyethylene oxide in the polymer component.

A variety of optional components and fillers also may be added to the films. These may include, without limitation: surfactants; plasticizers; polyalcohols; anti-foaming agents, such as silicone-containing compounds, which promote a smoother film surface by releasing oxygen from the film; thermo-setting gels such as pectin, carageenan, and gelatin, which help in maintaining the dispersion of components; inclusion compounds, such as cyclodextrins and caged molecules; coloring agents; and flavors. In some embodiments, more than one active ingredient may be included in the film.

Additives may be included in the films. Examples of classes of additives include excipients, lubricants, buffering agents, stabilizers, blowing agents, pigments, coloring agents, fillers, bulking agents, sweetening agents, flavoring agents, fragrances, release modifiers, adjuvants, plasticizers, flow accelerators, mold release agents, polyols, granulating agents, diluents, binders, buffers, absorbents, glidants, adhesives, anti-adherents, acidulants, softeners, resins, demulcents, solvents, surfactants, emulsifiers, elastomers and mixtures thereof. These additives may be added with the active agent(s).

Useful additives include, for example, gelatin, vegetable proteins such as sunflower protein, soybean proteins, cotton seed proteins, peanut proteins, grape seed proteins, whey proteins, whey protein isolates, blood proteins, egg proteins, acrylated proteins, water-soluble polysaccharides such as alginates, carrageenans, guar gum, agar-agar, xanthan gum, gellan gum, gum arabic and related gums (gum ghatti, gum karaya, gum tragancanth), pectin, water-soluble derivatives of cellulose: alkylcelluloses hydroxyalkylcelluloses and hydroxyalkylalkylcelluloses, such as methylcelulose, hydroxymethylcellulose, hydroxyethylcellulose, hydroxypropylcellulose, hydroxyethylmethylcellulose, hydroxypropylmethylcellulose, hydroxybutylmethylcellulose, cellulose esters and hydroxyalkylcellulose esters such as cellulose acetate phthalate (CAP), hydroxypropylmethylcellulose (HPMC); carboxyalkylcelluloses, carboxyalkylalkylcelluloses, carboxyalkylcellulose esters such as carboxymethylcellulose and their alkali metal salts; water-soluble synthetic polymers such as polyacrylic acids and polyacrylic acid esters, polymethacrylic acids and polymethacrylic acid esters, polyvinylacetates, polyvinylalcohols, polyvinylacetatephthalates (PVAP), polyvinylpyrrolidone (PVP), PVY/vinyl acetate copolymer, and polycrotonic acids; also suitable are phthalated gelatin, gelatin succinate, crosslinked gelatin, shellac, water-soluble chemical derivatives of starch, cationically modified acrylates and methacrylates possessing, for example, a tertiary or quaternary amino group, such as the diethylaminoethyl group, which may be quaternized if desired; and other similar polymers. Inventive films may further include compounds such as butylated hydroxytoluene.

Extenders may optionally be added in any desired amount desirably within the range of up to about 80%, desirably about 3% to 50% and more desirably within the range of 3% to 20% based on the weight of all film components.

Further additives may be inorganic fillers, such as the oxides of magnesium aluminum, silicon, titanium, etc. desirably in a concentration range of about 0.02% to about 3% by weight and desirably about 0.02% to about 1 % based on the weight of all film components.

Further examples of additives are plasticizers which include polyalkylene oxides, such as polyethylene glycols, polypropylene glycols, polyethylene-propylene glycols, organic plasticizers with low molecular weights, such as glycerol, glycerol monoacetate, diacetate or triacetate, triacetin, polysorbate, cetyl alcohol, propylene glycol, sorbitol, sodium diethylsulfosuccinate, triethyl citrate, tributyl citrate, and the like, added in concentrations ranging from about 0.5% to about 30%, and desirably ranging from about 0.5% to about 20% based on the weight of the polymer.

There may further be added compounds to improve the texture and/or flow properties of the starch material such as animal or vegetable fats, desirably in their hydrogenated form, especially those which are solid at room temperature. These fats desirably have a melting point of 50°C or higher. Preferred are tri-glycerides with C₁₂-, C₁₄-, C₁₆-, C₁₈-, C₂₀- and C₂₂- fatty acids. These fats can be added alone without adding extenders or plasticizers and can be advantageously added alone or together with mono- and/or di-glycerides or phosphatides, especially lecithin. The mono- and di-glycerides are desirably derived from the types of fats described above, i.e. with C₁₂-, C₁₄-, C₁₆-, C₁₈-, C₂₀- and C₂₂- fatty acids.

The total amounts used of the fats, mono-, di-glycerides and/or lecithins may be up to about 5% and preferably within the range of about 0.5% to about 2% by weight of the total film composition.

It further may be useful to add silicon dioxide, calcium silicate, or titanium dioxide in a concentration of about 0.02% to about 1 % by weight of the total composition. These compounds typically act as flow agents.

Lecithin is one surface active agent for use in the films described herein. Lecithin may be included in the feedstock in an amount of from about 0.25% to about 2.00% by weight. Other surface active agents, i.e. surfactants, include, but are not limited to, cetyl alcohol, sodium lauryl sulfate, the Spans™ and Tweens™ which are commercially available from ICI Americas, Inc. Ethoxylated oils, including ethoxylated castor oils, such as Cremophor® EL which is commercially available from BASF, are also useful. Carbowax™ is yet another modifier which is very useful in the present invention. Tweens™ or combinations of surface active agents may be used to achieve the desired hydrophilic-lipophilic balance ("HLB").

Other ingredients include binders which contribute to the ease of formation and general quality of the films. Non-limiting examples of binders include starches, pregelatinize starches, gelatin, polyvinylpyrrolidone, methylcellulose, sodium carboxymethylcellulose, ethylcellulose, polyacrylamides, polyvinyloxoazolidone, and polyvinylalcohols. If desired, the film may include other additives, such as keratin, or proteins, including proteins that are useful in forming a gel, such as gelatine.

Further potential additives include solubility enhancing agents, such as substances that form inclusion compounds with active ingredients. Such agents may be useful in improving the properties of very insoluble and/or unstable actives. In general, these substances are doughnut-shaped molecules with hydrophobic internal cavities and hydrophilic exteriors. Insoluble and/or instable actives may fit within the hydrophobic cavity, thereby producing an inclusion complex, which is soluble in water. Accordingly, the formation of the inclusion complex permits very insoluble and/or instable actives to be dissolved in water. A particularly desirable example of such agents are cyclodextrins, which are cyclic carbohydrates derived from starch. Other similar substances, however, are considered well within the scope of the present invention.

Suitable coloring agents include food, drug and cosmetic colors (FD&C), drug and cosmetic colors (D&C), or external drug and cosmetic colors (Ext. D&C). These colors are dyes, their corresponding lakes, and certain natural and derived colorants. Lakes are dyes absorbed on aluminum hydroxide.

Other examples of coloring agents include known azo dyes, organic or inorganic pigments, or coloring agents of natural origin. Inorganic pigments are preferred, such as the oxides or iron or titanium, these oxides, being added in concentrations ranging from about 0.001 to about 10%, and preferably about 0.5 to about 3%, based on the weight of all the components.

Flavors may be chosen from natural and synthetic flavoring liquids. An illustrative list of such agents includes volatile oils, synthetic flavor oils, flavoring aromatics, oils, liquids, oleoresins or extracts derived from plants, leaves, flowers, fruits, stems and combinations thereof. A non-limiting representative list of examples includes mint oils, cocoa, vanilla, creme flavors, and citrus oils such as lemon, orange, grape, lime and grapefruit and fruit essences including apple, pear, peach, grape, strawberry, raspberry, cherry, plum, pineapple, apricot or other fruit flavors.

Other useful flavorings include aldehydes and esters such as benzaldehyde (cherry, almond), citral i.e., alphacitral (lemon, lime), neral, i.e., beta-citral (lemon, lime), decanal (orange, lemon), aldehyde C-8 (citrus fruits), aldehyde C-9 (citrus fruits), aldehyde C-12 (citrus fruits), tolyl aldehyde (cherry, almond), 2,6-dimethyloctanol (green fruit), and 2-dodecenal (citrus, mandarin), combinations thereof and the like.

Inventive film compositions may further include sensates, such as cooling, tingling, or warming agents to provide a particular sensation to the user.

The sweeteners may be chosen from the following non-limiting list: glucose (corn syrup), dextrose, invert sugar, fructose, and combinations thereof; saccharin and its various salts such as the sodium salt; dipeptide sweeteners such as aspartame; dihydrochalcone compounds, glycyrrhizin; Stevia Rebaudiana (Stevioside); chloro derivatives of sucrose such as sucralose; polyols such as sorbitol, mannitol, xylitol, erythritol, and the like. Also contemplated are hydrogenated starch hydrolysates and the synthetic sweetener 3,6-dihydro-6-methyl-1-1-1,2,3-oxathiazin-4-one-2,2-dioxide, particularly the potassium salt (acesulfame-K), and sodium and calcium salts thereof, and natural intensive sweeteners, such as Lo Han Kuo. The films of the invention may further include sweeteners such as monoammonium glycyrrhizinate (sold under the trade name MagnaSweet). Other sweeteners may also be used.

The films may include one or more additives to aid in taste masking of the active ingredient. For example, the films may include ionic exchange resins, including but not limited to a water-insoluble organic or inorganic matrix material having covalently bound functional groups that are ionic or capable of being ionized under appropriate conditions. The organic matrix may be synthetic (e.g., polymers or copolymers or acrylic acid, methacrylic acid, sulfonated styrene or sulfonated divinylbenzene) or partially synthetic (e.g., modified cellulose or dextrans). The inorganic matrix may be, for example, silica gel modified by the addition of ionic groups. Most ion exchange resins are cross-linked by a crosslinking agent, such as divinylbenzene.

Anti-foaming and/or de-foaming components may also be used with the films. These components aid in the removal of air, such as entrapped air, from the film-forming compositions. Such entrapped air may lead to non-uniform films. Simethicone is one particularly useful anti-foaming and/or de-foaming agent. The present invention, however, is not so limited and other anti-foam and/or de-foaming agents may suitable be used.

As a related matter, simethicone and related agents may be employed for densification purposes. More specifically, such agents may facilitate the removal of voids, air, moisture, and similar undesired components, thereby providing denser, and thus more uniform films. Agents or components which perform this function can be referred to as densification or densifying agents. As described above, entrapped air or undesired components may lead to non-uniform films.

Simethicone is generally used in the medical field as a treatment for gas or colic in babies. Simethicone is a mixture of fully methylated linear siloxane polymers containing repeating units of polydimethylsiloxane which is stabilized with trimethylsiloxy endblocking unites, and silicon dioxide. It usually contains 90.5-99% polymethylsiloxane and 4-7% silicon dioxide. The mixture is a gray, translucent, viscous fluid which is insoluble in water.

When dispersed in water, simethicone will spread across the surface, forming a thin film of low surface tension. In this way, simethicone reduces the surface tension of bubbles air located in the solution, such as foam bubbles, causing their collapse. The function of simethicone mimics the dual action of oil and alcohol in water. For example, in an oily solution any trapped air bubbles will ascend to the surface and dissipate more quickly and easily, because an oily liquid has a lighter density compared to a water solution. On the other hand, an alcohol/water mixture is known to lower water density as well as lower the water's surface tension. So, any air bubbles trapped inside this mixture solution will also be easily dissipated. Simethicone solution provides both of these advantages. It lowers the surface energy of any air bubbles that trapped inside the aqueous solution, as well as lowering the surface tension of the aqueous solution. As the result of this unique functionality, simethicone has an excellent anti-foaming property that can be used for physiological processes (anti-gas in stomach) as well as any for external processes that require the removal of air bubbles from a product.

In order to prevent the formation of air bubbles in the films, the mixing step may be performed under vacuum. However, as soon as the mixing step is completed, and the film solution is returned to the normal atmosphere condition, air will be re-introduced into or contacted with the mixture. In many cases, tiny air bubbles will be again trapped inside this polymeric viscous solution. The incorporation of simethicone into the film-forming composition either substantially reduces or eliminates the formation of air bubbles during and after mixing.

Simethicone may be added to the film-forming mixture as an anti-foaming agent in an amount from about 0.01 weight percent to about 5.0 weight percent, more desirably from about 0.05 weight percent to about 2.5 weight percent, and most desirably from about 0.1 weight percent to about 1.0 weight percent.

Any other optional components described in commonly assigned U.S. Patent Nos. 7,425,292 and 7,357,891 and U.S. Application No. 10/856,176, referred to above, also may be included in the films described herein.

The active component or components in the film composition are desirably taste-masked, so as to prevent the user from the foul or bitter taste of the active. Further, since the active will remain in the mouth for an extended period of time (i.e., at least 30 seconds to 30 minutes), it is important that the active be sufficiently taste-masked for the time that the active is in the mouth. The Applicants have found that, as the film composition is dissolved in the mouth, some of the active components in the film are trapped on the user's tongue and slowly dissolve while on the tongue. As the active dissolves, it imparts a bitter and foul taste to the user. Thus, the present invention seeks to mask the foul taste perception of the active component. In particular, the foul taste is masked by providing a film composition which creates an alkaline micro-environment in the mouth. The alkaline environment reduces the solubility of many weakly basic active components, thereby reducing or altogether eliminating the bitter taste perception described above. In the case of ondansetron, ondansetron base is soluble at pH 1.2 and below. The solubility decreases with increasing pH becoming practically insoluble at pH 8.

The film compositions of the present invention include at least one active component. In some embodiments, the active component may be one which imparts a bitter taste to the user. To avoid the perception of a bitter taste to the user, the active composition may be taste masked as described herein. In some embodiments, the active component or components may have an acidic pH. In one particular embodiment the active to be masked includes ondansetron. However, the present method of taste masking can be applied to any active ingredient desired. The present invention is particularly effective at masking the foul taste associated with basic (alkaline) active compounds. Most particularly, effective compounds include tertiary amine compounds which are practically insoluble in water above pH 7.

Without limitation, exemplary actives that can be used in the present method include ace-inhibitors, antianginal drugs, anti-arrhythmias, anti-asthmatics, anti-cholesterolemics, analgesics, anesthetics, anti-convulsants, anti-depressants, antidiabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of medicating active ingredients contemplated for use in the present invention include antacids, H₂-antagonists, and analgesics. For example, antacid dosages can be prepared using the ingredients calcium carbonate alone or in combination with magnesium hydroxide, and/or aluminum hydroxide. Moreover, antacids can be used in combination with H₂-antagonists.

Analgesics include opiates and opiate derivatives, such as oxycodone (available as Oxycontin®), ibuprofen, aspirin, acetaminophen, and combinations thereof that may optionally include caffeine.

Other compounds that may be used in the present invention include anti-diarrheals such as Imodium AD, anti-histamines, anti-tussives, decongestants, vitamins, and breath fresheners. Common drugs used alone or in combination for colds, pain, fever, cough, congestion, runny nose and allergies, such as acetaminophen, chlorpheniramine maleate, dextromethorphan, pseudoephedrine HCl and diphenhydramine may be included in the film compositions of the present invention.

Also contemplated for use herein are anxiolytics such as alprazolam (available as Xanax®); anti-psychotics such as clozopin (available as Clozaril®) and haloperidol (available as Haldol®); non-steroidal anti-inflammatories (NSAID's) such as dicyclofenacs (available as Voltaren®) and etodolac (available as Lodine®), anti-histamines such as loratadine (available as Claritin®), astemizole (available as Hismanal™), nabumetone (available as Relafen®), and Clemastine (available as Tavist®); anti-emetics such as granisetron hydrochloride (available as Kytril®), serotonin 5-HT3 receptor antagonists (available as Ondansetron) and nabilone (available as Cesamet™); bronchodilators such as Bentolin®, albuterol sulfate (available as Proventil®); anti-depressants such as fluoxetine hydrochloride (available as Prozac®), sertraline hydrochloride (available as Zoloft®), and paroxtine hydrochloride (available as Paxil®); anti-migraines such as Imigra®, ACE-inhibitors such as enalaprilat (available as Vasotec®), captopril (available as Capoten®) and lisinopril (available as Zestril®); anti-Alzheimer's agents, such as nicergoline; and Ca^{H}-antagonists such as nifedipine (available as Procardia® and Adalat®), and verapamil hydrochloride (available as Calan®).

Erectile dysfunction therapies include, but are not limited to, drugs for facilitating blood flow to the penis, and for effecting autonomic nervous activities, such as increasing parasympathetic (cholinergic) and decreasing sympathetic (adrenersic) activities. Useful non-limiting drugs include sildenafils, such as Viagra®, tadalafils, such as Cialis®, vardenafils, apomorphines, such as Uprima®, yohimbine hydrochlorides such as Aphrodyne®, and alprostadils such as Caverject®.

The popular H₂-antagonists which are contemplated for use in the present invention include cimetidine, ranitidine hydrochloride, famotidine, nizatidien, ebrotidine, mifentidine, roxatidine, pisatidine and aceroxatidine.

The pharmaceutically active agents employed in the present invention may include allergens or antigens, such as , but not limited to, plant pollens from grasses, trees, or ragweed; animal danders, which are tiny scales shed from the skin and hair of cats and other furred animals; insects, such as house dust mites, bees, and wasps; and drugs, such as penicillin.

The film composition may also include at least one taste-masking component. Desirably, the taste-masking component is capable of at least partially preventing the active component from dissolving while in the oral cavity of the user. By at least partially preventing the active component from dissolving while in the user's oral cavity, the foul and bitter taste perception experienced by the user may be lessened or altogether prevented.

In some embodiments, particularly those incorporating a basic active compound, the taste-masking component may be a component that creates a basic environment in the mouth of the user. In this fashion, the taste-masking component may slow the dissolution of the active compound, thereby reducing or altogether eliminating the bitter taste associated with its dissolution. For example, the taste-masking component may include a basic composition such as calcium carbonate. The taste-masking component may further be a slow-dissolving component, so that it is maintained in the mouth of the user for an extended period of time, reducing the dissolution of the active component while the active component is also in the mouth. Thus, in some embodiments, the taste-masking component may be a slow-dissolving alkaline composition. In other embodiments, the taste-masking component may include a combination of at least one slow-dissolving alkaline component and at least one fast-dissolving alkaline component. Without limitation, suitable alkaline compositions include calcium carbonate, sodium bicarbonate, calcium stearate, calcium phosphate tribasic, aluminum hydroxide, magnesium hydroxide, and any other slow-dissolving alkaline material, particularly those having a pH greater than 7.

In one particularly useful embodiment, the composition includes at least one fast-dissolving alkaline component, such as sodium bicarbonate, and at least one slow-dissolving alkaline component, such as calcium carbonate. In this embodiment, the active is effectively taste-masked immediately upon insertion into the mouth of the user, since the fast-dissolving alkaline component begins to dissolve quickly. The composition is also effectively taste-masked over an extended period of time, since the slow-dissolving alkaline component is dissolved over the extended period of time.

The taste-masking component may be freely dispersed throughout the film composition, or it may be encapsulated. In some embodiments, the film composition may include both free taste-masking component(s) and encapsulated taste-masking components, so as to maintain the basic environment within the user's mouth for an extended period of time. Preferably, the taste-masking component is capable of producing a basic environment in the mouth for a period of at least 1 minute and may provide a basic environment in the mouth for up to 30 minutes. Most preferably, the basic environment is maintained in the mouth for at least the entire time that the film composition is dissolved in the mouth. During this period of time, the active component is released from the film composition, but is prevented from dissolving via the basic environment caused by the taste-masking component. Thus, for fast dissolving films, the basic environment should remain in the mouth for a period of at least 1 minute. For moderate-dissolving films, the basic environment should remain in the mouth for at least between 1 and 30 minutes. For slow-dissolving films, the basic environment should remain in the mouth for at least 30 minutes.

The taste-masking component may be present in any desired amount so as to achieve effective masking of the active component. In some embodiments, the taste-masking component(s) may be present in an amount of from about 0.1% to about 10% by weight of the composition. The taste-masking component(s) may alternatively be present in an amount of from 1 % to about 8% by weight of the composition. Desirably, the taste-masking component(s) are present in an amount of about 8% by weight of the composition. Any number of taste-masking components may be incorporated into the film composition. For example, in one embodiment, the composition may include about 8% calcium carbonate and about 1% sodium bicarbonate.

In some embodiments, the composition may include a multi-layered composition, including more than one film layer adhered or laminated together. The active(s) and taste-masking component(s) may be disposed in any layer, and may be disposed in separate layers. In such multi-layered embodiments, the first region may be dried prior to laminating any additional regions thereto. Similarly, the second region may be dried prior to laminating the first region thereto. Alternatively, either the first or second region may be at least partially dried prior to laminating any additional regions thereto.

### Clauses

The current invention can be described by the following clauses:
A self-supporting film dosage composition comprising: a polymer, an active component and at least one slow-dissolving alkaline component.
The composition of clause 1, wherein said slow-dissolving alkaline component is present in an amount of from about 0.1 % to about 10% by weight of the composition.
The composition of clause 1, wherein said slow-dissolving alkaline component comprises calcium carbonate.
The composition of clause 3, further comprising a fast-dissolving alkaline component.
The composition of clause 4, wherein said fast-dissolving alkaline component comprises sodium bicarbonate.
The composition of clause 4, wherein said slow-dissolving alkaline component is present in an amount of about 8% by weight of the composition and said fast-dissolving alkaline component is present in an amount of about 1 % by weight of said composition.
The composition of clause 6, wherein said slow-dissolving alkaline component comprises calcium carbonate and said fast-dissolving alkaline component comprises sodium bicarbonate.
The composition of clause 1, wherein said active component comprises ondansetron. The composition of clause 1, wherein said composition has a bitterness rating of from about 1 to about 2.

A self-supporting film dosage composition comprising:
An active component in an amount of about 10-15% by weight of said composition;
At least one slow-dissolving alkaline component in an amount of about 1-10% by weight of said composition; and
A polymeric matrix in an amount of about 75-89% by weight of said composition.

The composition of clause 10, wherein said slow-dissolving alkaline component comprises calcium carbonate.

The composition of clause 10, further comprising a fast-dissolving alkaline component. The composition of clause 12, wherein said fast-dissolving alkaline component comprises sodium bicarbonate.

The composition of clause 12, wherein said slow-dissolving alkaline component is present in an amount of about 8% by weight of the composition and said fast-dissolving alkaline component is present in an amount of about 1 % by weight of said composition.

The composition of clause 14, wherein said slow-dissolving alkaline component comprises calcium carbonate and said fast-dissolving alkaline component comprises sodium bicarbonate.

The composition of clause 10, wherein said active component comprises ondansetron. A method of making a self-supporting film dosage composition comprising the steps of: Providing a film-forming polymeric matrix;

Providing at least one active component;

Providing a slow-dissolving alkaline component;

Combining said film-forming polymeric matrix, said at least one active component and said slow-dissolving alkaline component to form an active matrix; and Drying said active matrix to form said self-supporting film dosage composition.

The method of clause 17, wherein said slow-dissolving alkaline component comprises calcium carbonate.

The method of clause 17, further comprising the step of providing a fast-dissolving alkaline component.

The method of clause 19, wherein said fast-dissolving alkaline component comprises sodium bicarbonate.

The method of clause 19, wherein said slow-dissolving alkaline component is present in an amount of about 8% by weight of the composition and said fast-dissolving alkaline component is present in an amount of about 1 % by weight of said composition.

The method of clause 21, wherein said slow-dissolving alkaline component comprises calcium carbonate and said fast-dissolving alkaline component comprises sodium bicarbonate.

The method of clause 17, wherein said active component comprises ondansetron.

### Examples

### Example 1 - Bitterness of Film Without Taste-Maskinq Component

An 8 mg film composition incorporating ondansetron was prepared and provided to a 5-person panel test to evaluate the bitterness perception of the film composition. The film composition is provided in Table 1 below:

**Table 1**

| **Component** | **Wt. Percentage (%)** |
|---|---|
| HPMC | 27.92 |
| PEO | 15.55 |
| Erythritol | 18.61 |
| Sucralose | 7 |
| Magnasweet | 0.5 |
| Xanthan gum | 0.5 |
| Silica | 1 |
| Ondansetron | 13.33 |
| Butylated hydroxytoluene | 0.1 |
| Flavors | 12.5 |
| Cooling agent | 1 |
| Prosweet | 1 |
| Titanium dioxide | 1 |
| Colorant | 0.03 |
| Total | 100 |

Each panelist placed the film strip in his or her mouth until dissolved. The panelist was then asked to evaluate the bitterness of the film strip on a scale of 1 to 5, with 1 being "no bitterness", 2 being "low bitterness", 3 being "medium bitterness", 4 being "too high bitterness", and 5 being "extremely high bitterness." The results are set forth in Table 2 below:

**Table 2**

| | **Panelist 1** | **Panelist 2** | **Panelist 3** | **Panelist 4** | **Panelist 5** |
|---|---|---|---|---|---|
| Bitterness Rating | 3.5 | 3.5 | 3.5 | 3.0 | 2.0 |

As can be seen, the film strip incorporating 8 mg of ondansetron without any taste-masking component registered an average bitterness rating of 3.10. A bitterness rating this high is between "medium bitterness" and "too high bitterness". This product therefore provided a bitter taste to the users, which would be considered unacceptable in terms of taste-masking.

### Example 2 - Bitterness of Film With Taste-Maskinq Component

An 8 mg film composition incorporating ondansetron and a taste-masking component was prepared and provided to a 5-person panel test to evaluate the bitterness perception of the film composition. In particular, the film composition included 8% calcium carbonate and 1% sodium bicarbonate. The film composition is provided in Table 3 below:

**Table 3**

| **Component** | **Wt. Percentage (%)** |
|---|---|
| HPMC | 23.87 |
| PEO | 13.26 |
| Erythritol | 15.91 |
| Sucralose | 7 |
| Magnasweet | 0.5 |
| Xanthan gum | 0.5 |
| Sodium bicarbonate | 1 |
| Silica | 1 |
| Calcium carbonate | 8 |
| Ondansetron | 13.33 |
| Butylated hydroxytoluene | 0.1 |
| Flavors | 12.5 |
| Cooling agent | 1 |
| Prosweet | 1 |
| Titanium dioxide | 1 |
| Colorant | 0.03 |
| Total | 100 |

Each panelist placed the film strip in his or her mouth until dissolved. The panelist was then asked to evaluate the bitterness of the film strip on a scale of 1 to 5, with 1 being "no bitterness", 2 being "low bitterness", 3 being "medium bitterness", 4 being "too high bitterness", and 5 being "extremely high bitterness." The results are set forth in Table 4 below:

**Table 4**

| | **Panelist 1** | **Panelist 2** | **Panelist 3** | **Panelist 4** | **Panelist 5** |
|---|---|---|---|---|---|
| Bitterness Rating | 1 | 1 | 1.5 | 2 | 2 |

As can be seen, the film strip incorporating 8 mg of ondansetron incorporating a taste masking component (8% calcium carbonate and 1% sodium bicarbonate) provided an average bitterness rating of 1.50, which is squarely between "no bitterness" and "low bitterness". A bitterness rating of this amount means that the active component is successfully taste-masked.

### Example 3 - Bitterness of Film Without Calcium Carbonate

An 8 mg film composition incorporating ondansetron and 1% sodium bicarbonate was prepared and provided to a 2-person panel test to evaluate the bitterness perception of the film composition. The film composition is provided in Table 5 below:

**Table 5**

| **Component** | **Wt. Percentage (%)** |
|---|---|
| HPMC | 27.47 |
| PEO | 15.26 |
| Erythritol | 18.31 |
| Sucralose | 7 |
| Magnasweet | 0.5 |
| Xanthan gum | 0.5 |
| Sodium bicarbonate | 1 |
| Silica | 1 |
| Ondansetron | 13.33 |
| Butylated hydroxytoluene | 0.1 |
| Flavors | 12.5 |
| Cooling agent | 1 |
| Prosweet | 1 |
| Titanium dioxide | 1 |
| Colorant | 0.03 |
| Total | 100 |

Each panelist placed the film strip in his or her mouth until dissolved. The panelist was then asked to evaluate the bitterness of the film strip on a scale of 1 to 5, with 1 being "no bitterness", 2 being "low bitterness", 3 being "medium bitterness", 4 being "too high bitterness", and 5 being "extremely high bitterness." The results are set forth in Table 6 below:

**Table 6**

| | **Panelist 1** | **Panelist 2** |
|---|---|---|
| Bitterness Rating | 2.5 | 4 |

As can be seen, the film strip incorporating 8 mg of ondansetron without calcium carbonate registered an average bitterness rating of 3.25. A bitterness rating of this level is found to provide a bitter taste to the user, which would be unacceptable in taste-masking.

### Example 4 - Bitterness of Film With 4% Calcium Carbonate

An 8 mg film composition incorporating ondansetron and a taste-masking component (including 4% calcium carbonate and 1% sodium bicarbonate) was prepared and provided to a 2-person panel test to evaluate the bitterness perception of the film composition. The film composition is provided in Table 7 below:

**Table 7**

| **Component** | **Wt. Percentage (%)** |
|---|---|
| HPMC | 25.67 |
| PEO | 14.26 |
| Erythritol | 17.11 |
| Sucralose | 7 |
| Magnasweet | 0.5 |
| Xanthan gum | 0.5 |
| Sodium bicarbonate | 1 |
| Silica | 1 |
| Calcium carbonate | 4 |
| Ondansetron | 13.33 |
| Butylated hydroxytoluene | 0.1 |
| Flavors | 12.5 |
| Cooling agent | 1 |
| Prosweet | 1 |
| Titanium dioxide | 1 |
| Colorant | 0.03 |
| Total | 100 |

Each panelist placed the film strip in his or her mouth until dissolved. The panelist was then asked to evaluate the bitterness of the film strip on a scale of 1 to 5, with 1 being "no bitterness", 2 being "low bitterness", 3 being "medium bitterness", 4 being "too high bitterness", and 5 being "extremely high bitterness." The results are set forth in Table 8 below:

**Table 8**

| | **Panelist 1** | **Panelist 2** |
|---|---|---|
| Bitterness Rating | 2 | 3 |

As can be seen, the film strip incorporating 8 mg of ondansetron with a taste-masking component of 4% calcium carbonate and 1% sodium bicarbonate provided a bitterness rating of 2.5. This rating indicates that the bitter taste was between "low" and "medium", which demonstrates an improvement over compositions lacking calcium carbonate. This composition would be considered successful in taste-masking the active. However, despite the successfulness of the taste-masking, this rating indicates that there is still some bitter taste associated with the composition.

### Example 5 - Bitterness of Film With 6% Calcium Carbonate

An 8 mg film composition incorporating ondansetron and a taste-masking component (including 6% calcium carbonate and 1% sodium bicarbonate) was prepared and provided to a 2-person panel test to evaluate the bitterness perception of the film composition. The film composition is provided in Table 9 below:

**Table 9**

| **Component** | **Wt. Percentage (%)** |
|---|---|
| HPMC | 24.77 |
| PEO | 13.76 |
| Erythritol | 16.51 |
| Sucralose | 7 |
| Magnasweet | 0.5 |
| Xanthan gum | 0.5 |
| Sodium bicarbonate | 1 |
| Silica | 1 |
| Calcium carbonate | 6 |
| Ondansetron | 13.33 |
| Butylated hydroxytoluene | 0.1 |
| Flavors | 12.5 |
| Cooling agent | 1 |
| Prosweet | 1 |
| Titanium dioxide | 1 |
| Colorant | 0.03 |
| Total | 100 |

Each panelist placed the film strip in his or her mouth until dissolved. The panelist was then asked to evaluate the bitterness of the film strip on a scale of 1 to 5, with 1 being "no bitterness", 2 being "low bitterness", 3 being "medium bitterness", 4 being "too high bitterness", and 5 being "extremely high bitterness." The results are set forth in Table 10 below:

**Table 10**

| | **Panelist 1** | **Panelist 2** |
|---|---|---|
| Bitterness Rating | 1.5 | 2 |

As can be seen, the film strip incorporating 8 mg of ondansetron with a taste-masking component of 6% calcium carbonate and 1% sodium bicarbonate provided an average bitterness rating of 1.75. This rating indicates that the bitter taste was between "none" and "low", which results in successfully taste-masking the active, and marks a considerable improvement over those compositions lacking calcium carbonate. The composition provided in this Example would be successful in reducing the bitter taste associated with the active.

### Example 6 - Bitterness of Film With 8% Calcium Carbonate

An 8 mg film composition incorporating ondansetron and a taste-masking component (including 8% calcium carbonate and 1% sodium bicarbonate) was prepared and provided to a 2-person panel test to evaluate the bitterness perception of the film composition. The film composition is provided in Table 11 below:

**Table 11**

| **Component** | **Wt. Percentage (%)** |
|---|---|
| HPMC | 23.87 |
| PEO | 13.26 |
| Erythritol | 15.91 |
| Sucralose | 7 |
| Magnasweet | 0.5 |
| Xanthan gum | 0.5 |
| Sodium bicarbonate | 1 |
| Silica | 1 |
| Calcium carbonate | 8 |
| Ondansetron | 13.33 |
| Butylated hydroxytoluene | 0.1 |
| Flavors | 12.5 |
| Cooling agent | 1 |
| Prosweet | 1 |
| Titanium dioxide | 1 |
| Colorant | 0.03 |
| Total | 100 |

Each panelist placed the film strip in his or her mouth until dissolved. The panelist was then asked to evaluate the bitterness of the film strip on a scale of 1 to 5, with 1 being "no bitterness", 2 being "low bitterness", 3 being "medium bitterness", 4 being "too high bitterness", and 5 being "extremely high bitterness." The results are set forth in Table 12 below:

**Table 12**

| | **Panelist 1** | **Panelist 2** |
|---|---|---|
| Bitterness Rating | 1 | **2** |

As can be seen, the film strip incorporating 8 mg of ondansetron with a taste-masking component of 8% calcium carbonate and 1% sodium bicarbonate provided an average bitterness rating of 1.5. This rating indicates that the bitter taste was between "none" and "low", which results in successfully taste-masking the active, and marks a considerable improvement over those compositions lacking calcium carbonate. The composition provided in this Example would be successful in reducing the bitter taste associated with the active.

## Claims

1. A self-supporting film dosage composition comprising: a polymer, an active component and at least one slow-dissolving alkaline component.

2. The composition of claim 1, wherein said slow-dissolving alkaline component is present in an amount of from about 0.1 % to about 10% by weight of the composition.

3. A self-supporting film dosage composition according to claim 1 comprising:
a. An active component in an amount of about 10-15% by weight of said composition;
b. At least one slow-dissolving alkaline component in an amount of about 1-10% by weight of said composition; and
c. A polymeric matrix in an amount of about 75-89% by weight of said composition.

4. A self-supporting film dosage composition comprising:
a. An active component in an amount of about 10-15% by weight of said composition;
b. At least one slow-dissolving alkaline component in an amount of about 1-10% by weight of said composition; and
c. A polymeric matrix in an amount of about 75-89% by weight of said composition.

5. The composition of any of the previous claims, wherein said slow-dissolving alkaline component comprises calcium carbonate.

6. The composition of any of the previous claims, further comprising a fast-dissolving alkaline component, preferably wherein said fast-dissolving alkaline component comprises sodium bicarbonate.

7. The composition of claim 6, wherein said slow-dissolving alkaline component is present in an amount of about 8% by weight of the composition and said fast-dissolving alkaline component is present in an amount of about 1 % by weight of said composition.

8. The composition of any of the claim 6 - 7, wherein said slow-dissolving alkaline component comprises calcium carbonate and said fast-dissolving alkaline component comprises sodium bicarbonate.

9. The composition of any of the previous claims, wherein said active component comprises ondansetron.

10. The composition of any of the previous claims, wherein said composition has a bitterness rating of from about 1 to about 2.

11. A method of making a self-supporting film dosage composition comprising the steps of:
a. Providing a film-forming polymeric matrix;
b. Providing at least one active component;
c. Providing a slow-dissolving alkaline component;
d. Combining said film-forming polymeric matrix, said at least one active component and said slow-dissolving alkaline component to form an active matrix; and
e. Drying said active matrix to form said self-supporting film dosage composition.

12. The method of claim 11, wherein said slow-dissolving alkaline component comprises calcium carbonate.

13. The method of any of the claims 11 - 12, further comprising the step of providing a fast-dissolving alkaline component, preferably wherein said fast-dissolving alkaline component comprises sodium bicarbonate.

14. The method of claim 13, wherein said slow-dissolving alkaline component is present in an amount of about 8% by weight of the composition and said fast-dissolving alkaline component is present in an amount of about 1 % by weight of said composition, preferably wherein said slow-dissolving alkaline component comprises calcium carbonate and said fast-dissolving alkaline component comprises sodium bicarbonate.

15. The method of any of the claims 11 -14, wherein said active component comprises ondansetron.
